# EUROPEAN PATENT APPLICATION

(11) **EP 4 603 572 A1**
(43) Date of publication of application: **20.08.2025**
(21) Application number: 24158226.1
(22) Date of filing: 16.02.2024
(51) Int. Cl.: C12M 1/00, C12M 3/06, C12M 3/00

(54) **SAMPLE CARTRIDGE**

(71) Applicant: Innotive Diagnostics Limited, Bath Somerset BA1 5BG (GB)
(72) Inventor: The designation of the inventor has not yet been filed
(74) Representative: Mewburn Ellis LLP

(57) **Abstract**

There is provided a sample cartridge for processing a fluid sample. The sample cartridge comprises a fluid pathway having an upstream end and a downstream end, wherein the fluid pathway is adapted to receive the fluid sample at the upstream end and route the fluid sample to the downstream end. The sample cartridge further comprises a sample chamber fluidically connected to the downstream end of the fluid pathway, the sample chamber comprising: a partition dividing the sample chamber into an upstream sub-chamber and a downstream sub-chamber, wherein the upstream sub-chamber is fluidically connected to the downstream end of the fluid pathway, and wherein the downstream sub-chamber is fluidically connected to the upstream sub-chamber via the partition. An absorptive element adapted to absorb at least part of the fluid sample is provided in the downstream sub-chamber.

## Description

### Field of the Invention

The present invention relates to sample cartridges and to sample chambers of said cartridges for processing a fluid sample and particularly, although not exclusively, to sample cartridges and sample chambers for processing a fluid sample containing bacteria.

### Background

Fluid sample processing and assessment is a key component of many medical tests. For example, in diagnosing infections, a fluid sample may need to be investigated for the presence of bacteria.

Numerous challenges arise when it comes to accurately and consistently assessing a fluid sample for the presence of bacteria, particularly when the assessment involves the visual investigation of the fluid sample. For instance, the number of bacteria present in a given fluid sample may be relatively low and the bacteria itself may be difficult to image, meaning that some means of encouraging bacterial growth to increase the number of bacteria, i.e., increase the bacterial concentration, in a sample and/or visual enhancement may be required.

However, it is often necessary to test several different means of encouraging bacterial growth and/or visual enhancement depending on the fluid sample and bacteria in question. Performing multiple such tests in order to identify the optimal combination is often laborious and complicated.

A further challenge arises when it comes to imaging the bacteria suspended in the fluid suspension due to the shallow depth of field of most microscopic imaging systems.

There exist several examples of cartridge-based fluid sample assessment systems. However, the cartridges often have a short shelf life, require particularly complicated active mechanisms for handling the fluid sample and/or are only able to perform a single test at a given time.

The present invention has been devised in light of the above considerations.

### Summary of the Invention

At its most general, the present invention provides a means of processing a fluid sample in order to assess the presence of bacteria within said fluid sample. The various aspects of the present invention improves over existing cartridge-based fluid sample assessment systems based on the considerations outlined above as well as others.

According to a first aspect of the invention, there is provided a sample cartridge for processing a fluid sample, the sample cartridge comprising: an inlet for receiving the fluid sample; a priming channel having an upstream priming end and a downstream priming end opposite the upstream priming end, wherein the priming channel is in fluid communication with the inlet at the upstream priming end of the priming channel; and a plurality of sample chambers, each sample chamber in fluid communication with the priming channel by way of one of a plurality of branch channels, each branch channel having an upstream branch end and a downstream branch end, wherein an upstream branch end of each of the branch channels is fluidically connected to the priming channel, between the upstream priming end and the downstream priming end of the priming channel, and wherein the downstream branch end of each of the branch channels is fluidically connected to a respective one of the sample chambers, and wherein the priming channel comprises a sealable vent located at the downstream priming end of the priming channel.

There is provided a sample cartridge for processing a fluid sample, and in particular a fluid sample containing bacteria. The cartridge includes an inlet for receiving the fluid sample, which is then provided to a priming channel.

The priming channel is fluidically connected, at an upstream priming end, to the fluid inlet and fluidically connected, at a downstream priming end, to a sealable vent, which is open when the priming channel has not been filled. As the fluid sample is provided to the fluid inlet, the priming channel will begin to fill with the fluid sample. As the priming channel fills, the fluid sample will flow from the upstream priming end to the downstream priming end forcing air in the priming channel out through the sealable vent. The sealable vent may be sealed when the priming channel has filled with the fluid sample or as the priming channel is filling with fluid sample.

The cartridge includes a plurality of sample chambers, each of which are connected to the priming channel by way of a respective branch channel. Each of the branch channels are connected to the priming channel at different positions between the fluid inlet and the sealable vent. Due to static pressures within the sample chambers, once the priming channel has filled with fluid sample sufficiently to pass a given branch channel, the filling of said branch channel will be resisted by the building air pressure therein and the priming channel will fill ahead of any of the branch channels because of the pressure release of the sealable vent. Due to dynamic pressures in the flowing fluid sample, there will be slight filling of the branch channels as the priming channel continues to fill.

Once the sealable vent is sealed, the pressure release of the sealable vent is no longer available to the priming channel and the branch channels will begin to fill at substantially similar times. In this way, the fluid sample may be processed in each of the plurality sample chambers substantially simultaneously.

The sample cartridge may be a cartridge for receiving and holding, or containing or storing, the fluid sample therein. The sample cartridge may be adapted to be received at, in or on, an apparatus for imaging the contents of the plurality sample chambers containing the fluid sample. At least one surface of the sample cartridge may be optically transparent. All of the surfaces of the sample cartridge may be adapted to allow at least some light to pass through. All of the internal surfaces of the sample cartridge, i.e., those surfaces in contact with the fluid sample may be biologically inert so as not to affect the bacteria held in the fluid sample. The sample cartridge may be any suitable cassette, magazine, canister, container, capsule or case for receiving and storing a fluid sample.

The fluid sample may be any liquid containing bacteria. For example, the fluid sample may comprise one or more of: urine; blood; saliva; and any other human or animal secretion.

The sample cartridge comprises a channel network for routing the fluid sample from the fluid inlet to the plurality of sample chambers. The priming channel is the channel of the sample cartridge that first receives the fluid sample from the fluid inlet. The branch channels are the channels of the sample cartridge that fluidically connect the priming channel to the sample chambers, each sample chamber being connected to the priming channel by a single branch channel.

The fluid sample flows along the priming channel and to the sealable vent first, and then, when the sealable vent is sealed, to the branch channels and the sample chambers. Put another way, the fluid sample does not flow to the sample chambers until after the priming channel has been primed, i.e., filled with fluid sample.

In some examples, the sealable vent is a hydrophobic vent, and wherein the hydrophobic vent is gas permeable in a dry state, and wherein the hydrophobic vent is sealed in a wet state.

The hydrophobic vent is gas permeable when dry and is liquid impermeable up to a predetermined pressure threshold. The predetermined pressure threshold may be higher than pressures within the cartridge during use. The hydrophobic vent may be any suitable hydrophobic material, such as a hydrophobic paper or permeable membrane.

In this way, there is provided a cartridge for receiving a fluid sample and routing the fluid sample to a plurality of sample chambers in the same filling action, without requiring further input. Put another way, there is provided a sample cartridge adapted to automatically fill a plurality of sample chambers with a received fluid sample, without requiring any intervention other than providing the fluid sample to an inlet.

When the sample cartridge is dry, i.e., before any fluid sample has been introduced to the fluid inlet, the channel network is filled with air. As the fluid sample is introduced to the priming channel, via the fluid inlet, the air in the channel network is displaced. The hydrophobic vent is the only external outlet in the sample cartridge, meaning that the hydrophobic vent is the only point at which air in the sample cartridge may escape and thereby release pressure. As the fluid sample flows along the priming channel, the fluid sample is effectively sealing each of the branch channels and sample chambers with air still inside, creating a pressure differential between the sample chambers and the priming channel. This pressure differential will cause the priming channel to fill first, due to the continue pressure release of the hydrophobic vent, until the hydrophobic vent is sealed and the pressure in the priming channel begins to build, causing the fluid sample to begin flowing along the branch channels towards the sample chambers.

Alternatively, the sealable vent may be any other suitable sealable vent for sealing the priming channel, such as: a mechanical vent; a pneumatic vent; and the like.

In some examples, each of the plurality of branch channels comprises a branch inlet section extending from the upstream branch end of the branch channel to a downstream branch inlet end, and wherein an angle between a priming flow vector, the priming flow vector being defined from the upstream priming end to the downstream priming end of the priming channel, and a branch flow vector, the branch flow vector being defined from the upstream branch end to the downstream branch inlet end, is an acute angle.

Put another way, the plurality of branch channels may each connect to the priming channel at an angle, and more specifically, at an angle that changes the direction of flow of the fluid sample. In other words, the transition between the priming channel and the branch channel may force a change in the direction of flow of the fluid sample in order to reduce the dynamic pressure effects that would cause the branch channels to fill before the priming channel has been filled.

In this way, the branch channel may be prevented from filling before the priming channel has been filled due to the additional pressure required to cause the fluid sample to change flow direction and fill the branch channels.

In some examples, each of the plurality of branch channels comprises at least one bend between the upstream branch end and the downstream branch end of the branch channel, and optionally wherein each of the plurality of branch channels is routed such that fluid sample flowing along a portion of each branch channel flows in a direction opposite to fluid sample flowing in the priming channel.

In this way, the pressure required to fill the branch channel may be further increased in order to ensure the sample chambers do not fill before the priming channel has been filled with the fluid sample. By providing a bend in the branch channels, even if some fluid sample enters the branch channels before the priming channel has been completely filled due to the dynamic pressure of the flowing fluid sample, the downstream branch end may be distanced from the upstream branch end of the branch channel to prevent fluid sample from entering the sample chamber itself.

Thus, the channel network of the sample cartridge may be arranged in order to control the flow of the fluid sample without requiring any active components. Put another way, the distribution of fluid sample from the fluid inlet to the sample chambers may be controlled passively based on pressure differentials alone, thereby reducing the complexity of the sample cartridge, and reducing the possible points of failure within the cartridge itself, thereby increasing the reliability of the sample cartridge.

In some examples, at least one of the plurality of branch channels further comprises an analyte chamber between the upstream branch end and the downstream branch end, wherein the analyte chamber comprises a reagent to be mixed with the fluid sample as the fluid sample flows through the analyte chamber.

In this way, reagents may be introduced to the fluid sample as it flows through the sample cartridge from the priming channel to the sample chambers via the branch channels. By providing the analyte chamber along the branch channels, as opposed to the priming channel, it is ensured that the sample proportion of analyte may be provided to the fluid sample received in each sample chamber. Thus, each portion of the fluid sample received in the respective sample chambers may be received at the same time and with a controlled amount of reagent mixed in, thereby providing multiple consistent testing environments within a single sample cartridge. The controlled amount of reagent may be the same across all, or some, of the analyte chambers. Alternatively, each reagent chamber may contain different amounts of the same reagents, different reagents or no reagent.

In some examples, reagents may be provided outside of the analyte chamber elsewhere within the sample cartridge for mixing with the fluid sample. For example, reagents may be provided in the branch channel, such as between the analyte chamber and the sample chamber.

In some examples, the analyte chamber is provided downstream of the at least one bend.

In this way, the distance between the point at which the branch channel is joined to the priming channel and the reagents may be controlled to ensure that the reagents in the analyte chamber are not introduced to the fluid sample until the fluid sample has begun distributed to all of the sample chambers, i.e., after the priming channel has been filled. Accordingly, unintentional and premature mixing of the reagent with the fluid sample may be avoided.

In some examples, the analyte chamber comprises one or more mixing structures adapted to generate turbulence in a flow of fluid sample through the analyte chamber.

In this way, the reagent may be more evenly distributed and mixed within the fluid sample as it travels from the priming channel to the given sample chamber.

In some examples, the plurality of sample chambers comprises: a first row of sample chambers provided on a first side of the priming channel; and a second row of sample chambers provided on a second, opposing side of the priming channel to the first row. Put another way, the sample chambers may be arranged on either side of the priming channel.

In some examples, the upstream branch ends of the plurality of branch channels fluidically connecting the first row of sample chambers to the priming channel are aligned with the upstream branch ends of the plurality of branch channels fluidically connecting the second row of sample chambers to the priming channel.

In this way, the channel network, and in particular the sample chambers and the branch channels, may be symmetrical along the line of the priming channel. In this way, the manufacture of the sample cartridge may be simplified.

In some examples, the upstream branch ends of the plurality of branch channels fluidically connecting the first row of sample chambers to the priming channel are offset, along the length of the priming channel, from the upstream branch ends of the plurality of branch channels fluidically connecting the second row of sample chambers to the priming channel.

In this way, the branch channels may be offset along the length of the priming channel. For example, the upstream branch ends of the branch channels connecting the first row of branch channels to the priming channel may be interleaved with the upstream branch ends of the branch channels connecting the second row of branch channels to the priming channel. Alternatively, the upstream branch ends of the branch channels may be offset at either side of the priming channels at another regular, or irregular, interval. In this way, the number of sample chambers that can be provided within the sample chamber may be tuned or maximised.

In some examples, the sample cartridge further comprises a plurality of gas spring arrangements, wherein each gas spring arrangement is fluidically connected to a respective one of the plurality of sample chambers downstream of said respective sample chamber.

A gas spring arrangement, or a gas spring, may be a chamber having only a single inlet/outlet and containing only gas, or air. As air is a compressible fluid, when a chamber or channel fluidically connected to the gas spring is filled, for example with a fluid sample, the air within the gas spring arrangement will be compressed and the pressure within the gas spring arrangement will increase.

When the priming channel is filled with the fluid sample, and the sealable vent is sealed, the sample cartridge becomes a pressure sealed unit. At this point, the gas spring arrangements connected to each of the sample chambers will cause the pressure acting against the fluid sample across all of the branch channels to equalise before all of the branch channels begin to fill equally. Therefore, the provision of the gas spring arrangements balance the filling of the sample chambers such that each sample chamber is filled at substantially the same time, or simultaneously.

The volume of the gas spring arrangements, and in particular the volume of the gas spring arrangements relative to the air being displaced when the fluid sample is introduced to the sample cartridge, governs the pressure along each respective branch channel. For example, a gas spring arrangement having a larger volume will generate a lower pressure compared to a gas spring arrangement with a smaller volume in branch channels having equivalent dimensions. Having lower pressures within the sample cartridge may reduce the risk of the sample cartridge leaking during or after testing of the fluid sample.

In some examples, each of the gas spring arrangements may have the same volume. In other examples, each of the gas spring arrangements may have different volumes. In some cases, the gas spring arrangements may be divided into one or more subsets of gas spring arrangements, wherein each subset of gas spring arrangements has a different volume, but each gas spring arrangement within a given subset of gas spring arrangements has the same volume. The volume of each gas spring arrangement may be adjusted in order to control the filling rate of, and/or the volume of fluid sample received at, the respective sample chamber as desired.

By way of an example, the different gas spring arrangement volumes may be used to control the reagent concentration in the fluid sample as it reaches the sample chamber, by controlling the volume of fluid sample that is mixed in analyte chambers having the same amount of reagent present. In another example, where a fluid sample is expected to have different concentrations of pathogens present, the different gas spring arrangement volumes may be used to distribute the fluid sample to sample chambers at a volume appropriate for the different concentrations of pathogens, for example a lower volume of fluid sample may be provided to a sample chamber for a high concentration of a pathogen and a higher volume of fluid sample may be provided to a sample chamber for a low concentration of another pathogen.

The gas spring arrangements may cause the fluid sample to oscillate slightly between the downstream end of the branch channel and the sample chamber. In the case where reagents are provided in the branch channel between the sample chamber and the analyte chamber, i.e., towards the upstream end of the branch channel, the oscillation, or backflow, in the fluid sample may be leveraged to cause the reagent to be mixed with the fluid sample.

In some examples, each sample chamber comprises a partition dividing each sample chamber into an upstream sub-chamber and a downstream sub-chamber, wherein the upstream sub-chamber is fluidically connected to the downstream branch end of the branch channel, and wherein the downstream sub-chamber is fluidically connected to the upstream sub-chamber via the partition.

In this way, each sample chamber may be filled in stages with the upstream sub-chamber filling before the downstream sub-chamber. By sub-dividing each of the sample chambers with a partition in this manner, an additional pressure threshold is introduced in order for the fluid sample to reach the downstream sub-chamber of each sample chamber, meaning that the upstream sub-chamber of each of the sample chamber may be filled prior to the filling of the downstream sub-chamber. Accordingly, the filling of the sample chambers may be controlled with greater accuracy without requiring any additional active components to be added to the sample cartridge.

In some examples, each gas spring arrangement comprises: a priming gas spring fluidically connected to the upstream sub-chamber; and a balancing gas spring fluidically connected to the downstream sub-chamber.

Put another way, the gas spring arrangement may be a two-stage gas spring adapted to control the filling of each sub-chamber the given sample chamber. In particular, the priming gas spring may regulate the pressure within the branch channel and the upstream sub-chamber as the upstream sub-chamber fills with the fluid sample, which may result in a reduction in the amount, or number, of bubbles that form or get trapped between the fluid sample and the partition. In other words, the priming gas spring may provide somewhere for any air trapped in the fluid sample to go before the fluid sample enters the downstream sub-chamber.

Once the upstream sub-chamber has been filled sufficiently, and the pressure in the priming gas spring has reached a predetermined threshold, the fluid sample may be forced into the downstream sub-chamber through the partition. The nature and function of the partition is discussed in further detail below.

The balancing gas springs may control the rate of filling of the downstream sub-chambers across all of the sample chambers by balancing the pressure difference between the upstream sub-chamber and the downstream sub-chamber in each sample chamber.

It should be noted that whilst the priming gas spring and the balancing gas spring are separate, both will act on the fluid sample as it travels along the priming channel the branch channels and begins to fill the upstream sub-chamber. Put another way, at the initial stages of filling the sample cartridge, the priming gas spring and the balancing gas spring may act as a single gas spring. It is not until the priming gas spring reaches a state of maximum compression that the balancing gas springs will become the sole gas spring controlling the filling of all of the sample chambers to completion.

In some examples, the balancing gas spring is fluidly connected to the downstream sub-chamber by a plurality of ducts, and wherein the balancing gas spring comprises a plurality of gas spring chambers fluidically connected to each other, and wherein each of the plurality of ducts is fluidically connected to a respective one of the plurality of gas spring chambers.

By providing multiple ducts connecting the balancing gas spring and the downstream sub-chamber, the fluidic connections between the balancing gas spring and the sample chamber may be provided with redundancy in order to reduce the likelihood of the fluidic connection between the balancing gas spring and the sample chamber becoming blocked and disabling the balancing gas spring.

According to a second aspect of the invention, there is provided a method for filling a sample chamber of a sample cartridge with a fluid sample, the method comprising: providing a fluid sample to an inlet of the sample cartridge, thereby filling a priming channel of the sample cartridge, the priming channel having an upstream priming end and a downstream priming end opposite the upstream priming end, wherein the priming channel is fluidically connected to the inlet at the upstream priming end of the priming channel; sealing a sealable vent at the downstream priming end of the priming channel when the priming channel is filled with the fluid sample; continuing to provide the fluid sample to the inlet of the sample cartridge, thereby causing the fluid sample to flow through a plurality of branch channels, which fluidically connect to the priming channel at an upstream branch end of each branch channel and to a sample chamber at a downstream branch end of each branch channel, thereby filling a plurality of sample chambers.

In some examples, the sample cartridge further comprises a plurality of gas spring arrangements fluidically connected to one of the plurality of sample chambers downstream of said sample chamber, and wherein the method further comprises: dynamically balancing the fluid flow along the plurality of branch channels and into the plurality of sample chambers by compressing air within the plurality of gas spring arrangements as the fluid sample flows along the plurality of branch channels and towards the plurality of sample chambers, and optionally wherein the method further comprises filling the plurality of sample chambers substantially simultaneously by dynamically balancing the fluid flow along the plurality of branch channels and into the plurality of branch chambers.

According to a third aspect of the invention, there is provided a sample cartridge for processing a fluid sample, the sample cartridge comprising: an inlet for receiving the fluid sample; a main channel having an inlet end fluidically connected to the inlet; a plurality of sample chambers, each sample chamber fluidically connected to the main channel by way of one of a plurality of branch channels; and a plurality of gas spring arrangements, each gas spring arrangement being fluidically connected to one of the plurality of sample chambers, wherein each gas spring arrangement comprises: an upstream gas spring fluidically connected to the sample chamber at a first position; and a downstream gas spring fluidically connected to the sample chamber at a second position different to the first position.

There is provided a sample cartridge for processing a fluid sample, and in particular a fluid sample containing bacteria. The cartridge includes an inlet for receiving the fluid sample, which is then provided to a main channel.

The main channel is fluidically connected, at an upstream end, to the fluid inlet and fluidically connected to a plurality of sample chambers, each of which are connected to the main channel by way of a respective branch channel. In other words, there is provided a cartridge for receiving a fluid sample and routing the fluid sample to a plurality of sample chambers in the same filling action, without requiring further input.

Put another way, there is provided a sample cartridge adapted to automatically fill a plurality of sample chambers with a received fluid sample, without requiring any intervention other than providing the fluid sample to an inlet.

As outlined above, a gas spring arrangement, or a gas spring, may be a chamber having only a single inlet/outlet and containing only gas, or air. As air is a compressible fluid, when a chamber or channel fluidically connected to the gas spring is filled, the air within the gas spring arrangement will be compressed and the pressure within the gas spring arrangement will increase.

When the main channel is filled with the fluid sample and the fluid sample begins to flow along a branch channel, the given branch channel and connected sample chamber and gas spring arrangement becomes a pressure sealed unit. At this point, the gas held in the given gas spring arrangement will cause the pressure acting against the fluid sample in the branch channel to increase and the fluid sample will preferentially flow along the main channel until all of the branch channel pressures have equalised, at which point the branch channels begin to fill equally. Therefore, the provision of the gas spring arrangements balance the filling of the sample chambers such that each sample chamber is filled at substantially the same time, or simultaneously.

The gas spring arrangement is a two-stage gas spring adapted to control the filling of each sample chamber in stages. In particular, when the upstream gas spring reaches a state of maximum compression, the downstream gas spring will continue to control the filling of all of the sample chambers to completion. In this way, the control of the filling of the sample chambers may be made more accurate whilst only using passive elements.

According to a fourth aspect of the invention, there is provided a sample cartridge for processing a fluid sample, the sample cartridge comprising: a fluid pathway having an upstream end and a downstream end, wherein the fluid pathway is adapted to receive the fluid sample at the upstream end and route the fluid sample to the downstream end; a sample chamber fluidically connected to the downstream end of the fluid pathway, the sample chamber comprising: a partition dividing the sample chamber into an upstream sub-chamber and a downstream sub-chamber, wherein the upstream sub-chamber is fluidically connected to the downstream end of the fluid pathway, and wherein the downstream sub-chamber is fluidically connected to the upstream sub-chamber via the partition; and an absorptive element adapted to absorb at least part of the fluid sample, wherein the absorptive element is provided in the downstream sub-chamber.

There is provided a sample cartridge for processing a fluid sample, and in particular a fluid sample containing bacteria. The cartridge includes a fluid pathway for receiving the fluid sample and routing to a sample chamber. The sample cartridge may include one or more, or all, of the aspects described above. For example, the fluid pathway may include the priming channel, or main channel, and the branch channels described above and the sample chamber may include one or more of the sample chambers described above.

The sample cartridge may be a cartridge for receiving and holding, or containing or storing, the fluid sample therein. The sample cartridge may be adapted to be receiving at, in or on, an apparatus for imaging the contents of the plurality sample chambers containing the fluid sample. At least one surface of the sample cartridge may be optically transparent. All of the surfaces of the sample cartridge may be adapted to allow at least some light to pass through. All of the internal surfaces of the sample cartridge, i.e., those surfaces in contact with the fluid sample may be non-reactive so as not to affect the bacteria held in the fluid sample. The sample cartridge may be any suitable cassette, magazine, canister, container, capsule or case for receiving and storing a fluid sample.

The fluid sample may be any liquid containing bacteria. For example, the fluid sample may comprise one or more of: urine; blood; saliva; and any other human or animal secretion.

The sample chamber is divided, or partitioned, into an upstream sub-chamber, which is directly fluidically connected to the fluid pathway, and a downstream sub-chamber, which is fluidically connected to the upstream sub-chamber via, or through, the partition.

In this way, the sample chamber may be filled in stages with the upstream sub-chamber filling before the downstream sub-chamber. By sub-dividing the sample chamber with a partition in this manner, an additional pressure threshold is introduced in order for the fluid sample to reach the downstream sub-chamber, meaning that the upstream sub-chamber of each of the sample chamber may be filled prior to the filling of the downstream sub-chamber. Accordingly, the filling of the sample chambers may be controlled with greater accuracy without requiring any additional active components to be added to the sample cartridge.

The absorptive element is provided in the downstream sub-chamber of the sample chamber. The absorptive element may comprise a hydrogel. The absorptive element does not begin to absorb the fluid sample until the fluid sample begins to pass through the partition and into the downstream sub-chamber, meaning that the upstream sub-chamber has the opportunity to fill before the absorption of the fluid sample begins.

By positioning the absorptive element in the downstream sub-chamber of the sample chamber, separated from the upstream sub-chamber by way of a partition, the amount of fluid sample held in the sample chamber may be accurately controlled.

In other words, there is provided a cartridge for receiving a fluid sample and maintaining an accurate amount of fluid sample in the sample chamber in the same filling action, without requiring further input.

Put another way, there is provided a sample cartridge adapted to automatically fill a sample chamber with a desired amount of fluid sample, without requiring any intervention other than providing the fluid sample to an inlet of the fluid pathway.

In some examples, the absorptive element is three-dimensional, i.e., not planar. Put another way, the absorptive element may be provided in a format that is not sheet-like. For example, the absorptive element may be spherical, or substantially spherical or rounded. In an example, the absorptive element may be provided in the form of a ball of hydrogel.

In some examples, the absorptive element comprises a dissolvable coating to adjust an absorption rate of the fluid sample by the absorptive element.

For example, the dissolvable coating may retard the absorption of the fluid sample by the absorptive element. In this way, the downstream sub-chamber may at least partially fill with the fluid sample before the absorptive element begins to absorb the fluid sample. Accordingly, the delay in the absorbing of the fluid sample may prevent the fluid sample from being absorbed prematurely, for example, before the upstream sub-chamber has filled fully, or to a desired point or for a desired length of time.

In another example, the dissolvable coating may accelerate the absorption of the fluid sample by the absorptive element. In this way, the absorption of the fluid sample, and any associated changes in volume of the absorptive element and any results of this change in volume (examples of which are described further below), may be accelerated.

In some examples: in the absence of the fluid sample in the downstream sub-chamber, the absorptive element is in a dehydrated state; and in the presence of the fluid sample in the downstream sub-chamber, the absorptive element is adapted to absorb at least part of the fluid sample to change from the dehydrated state to a hydrated state, and wherein, a volume of the absorptive element in the hydrated state is larger than a volume of the absorptive element in the dehydrated state.

Put another way, the absorptive element may swell on the absorption of the fluid sample in order to change in size. In other words, a greater proportion of the downstream-sub chamber may be occupied by the absorptive element in its hydrated state compared to the absorptive element in its dehydrated state.

In this way, the pressure within the sample chamber and the fluid pathway may be controlled, and in particular prevented from falling, as the fluid sample in the downstream sub-chamber is absorbed.

In some examples, the absorptive element is movably received in the downstream sub-chamber when the absorptive element is in the dehydrated state. Put another way, the absorptive element may not be fixed, or held, at a particular position in the downstream sub-chamber. In this way, the absorptive element is free to move as it absorbs the fluid sample passing through the partition and may be free to move and expand as it changes from the dehydrated state to the hydrated state. Accordingly, the absorptive element may be prevented from ceasing to absorb the fluid sample due to, for example, becoming trapped or stuck in a position that does not allow for more expansion to occur.

In some examples, a volume of the downstream sub-chamber is lower than the volume of the absorptive element in the hydrated state, such that the absorptive element in the hydrated state exerts a force on the partition.

Put another way, in the hydrated state, the absorptive element may be larger than the downstream sub-chamber. In this way, the downstream sub-chamber may be filled with the absorptive element after a sufficient quantity of the fluid sample has been absorbed. Accordingly, the amount of fluid sample held in the sample chamber may be accurately controlled.

In some examples, the partition is flexible such that the partition deforms under the force exerted by the absorptive element in the hydrated state.

In this way, the shape of the partition may be altered by the absorptive element, and in particular by the expansion of the absorptive element. For example, the expanding absorptive element may push the partition into the upstream sub-chamber. Accordingly, the amount of fluid sample held in the sample chamber may be controlled in a further manner by the absorptive element and the deformation of the partition.

In some examples, the partition is adapted to become transparent on contact with the fluid sample.

In this way, bacterial growth may be imaged through the partition without impacting the image quality obtained, thereby providing more degrees of freedom for orienting the sample chamber in an imaging system.

In addition, if the partition were not transparent until rehydration, there would be provided an additional visual indication of whether the sample chamber contained, or has recently, contained fluid. In this way, it can be assessed whether a sample chamber has not filled properly or if a sample chamber has already been used.

In some examples, the upstream sub-chamber comprises a bacterial growth platform. Aspects of the bacterial growth platform are discussed in further detail below; however, the bacterial growth platform may be any platform or material suitable for encouraging the growth of bacteria. By providing a bacterial growth platform within the sample chamber, bacteria present in the fluid sample may be encouraged to grow in number on the bacterial growth platform. Bacterial growth may then be measured, and in some cases compared to a control sample chamber (which may be a sample chamber of the sample cartridge absent of any reagent and/or bacterial growth platform) over time in order to derive clinical information.

In some examples, the partition comprises a fluid permeable filter adapted to prevent the passage of bacteria through the filter, the bacteria being present in the fluid sample. Put another way, as the fluid sample flows through the partition from the upstream sub-chamber to the downstream sub-chamber, the bacteria present in the fluid sample may be captured on the partition. In this way, bacteria present in the fluid sample may be concentrated to a given position within the sample chamber.

When imaging a sample chamber to investigate the presence of bacteria within the fluid sample, the imaging systems used typically have a very shallow depth of field. This means that imaging bacteria suspended freely within a fluid is extremely difficult to perform with any accuracy.

By concentrating the bacteria at a known location within the sample chamber, such as the partition, the bacteria may be more readily and accurately imaged.

In some examples, a volume of the sample chamber is lower than the volume of the absorptive element in the hydrated state, such that the partition is deformed to contact the bacterial growth platform.

In this way, the bacteria captured at the partition may be forced into contact with the bacterial growth platform, thereby ensuring that any bacteria present in the fluid sample will be cultured within the sample chamber and so improving the accuracy of testing the fluid sample with the sample cartridge.

In some examples, the partition is deformed to be substantially flattened against the bacterial growth platform.

In this way, the bacteria present in the fluid sample is concentrated in one place, i.e., the partition, of the sample chamber, which is then forced into contact with the bacterial growth platform in order to encourage said concentrated bacteria to grow and flattened in order to keep the bacteria in a single plane for accurate imaging. Further, when the partition is flattened against the bacterial growth platform, the partition clamps the bacteria in place, preventing any lateral movement, i.e., movement in the plane perpendicular to the movement of the partition towards the bacterial growth platform, from occurring, thereby further improving the imaging accuracy that can be achieved using the sample cartridge.

In some examples, the fluid pathway widens along a downstream direction as it approaches the sample cartridge from an upstream direction.

In this way, the number of bubbles present in the upstream sub-chamber may be reduced as the upstream sub-chamber may fill more completely before the partition is wetted by the fluid sample, which may cause pockets of air to become trapped in the upstream sub-chamber.

According to a fifth aspect of the invention, there is provided a method for filling a sample chamber of a sample cartridge with a fluid sample, the sample chamber comprising a partition dividing the sample chamber into an upstream sub-chamber and a downstream sub-chamber, and wherein the sample chamber comprises an absorptive element provided in the downstream sub-chamber adapted to absorb at least part of the fluid sample, the method comprising: providing a fluid sample to a fluid pathway of the sample cartridge, thereby filling an upstream sub-chamber of the sample chamber that is fluidically connected to a downstream end of the fluid pathway; and continuing to provide the fluid sample to the fluid pathway of the sample cartridge, thereby causing the fluid sample to flow through the partition from the upstream sub-chamber to the downstream sub-chamber.

In some examples, the partition is flexible and the upstream sub-chamber comprises a bacterial growth platform, and wherein the method further comprises: deforming the partition to contact the bacterial growth platform, wherein deforming the partition comprises: continuing to provide the fluid sample to the fluid pathway of the sample cartridge, thereby causing the fluid sample to be absorbed by the absorptive element, such that the absorptive element changes from a dehydrated state to a hydrated state, wherein a volume of the absorptive element in the hydrated state is larger than a volume of the sample chamber such that the absorptive element in the hydrated state exerts a force on the partition, thereby deforming the partition to contact the bacterial growth platform.

In some examples, the method further comprises continuing to supply fluid sample to the fluid pathway of the sample cartridge, thereby providing a flow of fluid sample to the absorptive element as the fluid sample in the sample chamber is absorbed. In this way, the absorptive element is prevented from drying out the other components of the sample chamber, such as the partition or the bacterial growth platform.

According to a sixth aspect of the invention, there is provided a sample chamber for receiving a fluid sample, wherein the sample chamber comprises: a dehydrated bacterial growth platform adapted to rehydrate on contact with the fluid sample to encourage bacterial growth in the sample chamber, the bacteria being present in the fluid sample.

There is provided a sample chamber for receiving a fluid sample and culturing bacteria present in the fluid sample. The sample chamber may be provided on any of the sample cartridges described above. The sample chamber may include any of the features described above with reference to the sample chambers of the sample cartridges described.

As discussed below, a bacterial growth platform may be any material suitable for bacterial survival. The bacterial growth platform may comprise a combination of a bacterial growth medium and a gelling agent.

Typically, bacterial growth platforms are hydrated due to the fact that bacterial growth requires the presence of water or moisture. Nutrients may be added to a fluid sample in order to encourage bacterial growth therein; however, this is often not suitable in imaging applications where imaging bacteria in fluid suspension is difficult to perform with any accuracy, due to the movement of bacteria and the small working distance of the microscope objective lens at higher magnification levels. In laboratory settings, bacterial growth platforms are prepared fresh as needed or can be stored with a relatively short shelf life. If such a bacterial growth platform were to dehydrate and degrade, or dry out sufficiently, it would ordinarily be disposed of.

However, the present inventors have discovered that a dehydrated bacterial growth platform can be used to encourage bacterial growth from bacteria present in a fluid sample due to the rehydration of the bacterial growth platform by said fluid sample.

In addition, the use of a dehydrated bacterial growth platform may elongate the shelf life of the sample cartridge due to the fact that any nutrients for encouraging bacterial growth in the dehydrated bacterial growth platform are essentially preserved.

In some examples, the dehydrated bacterial growth platform comprises a dehydrated gel medium, such as dehydrated agar or agarose.

Agar and agarose are commonly used in laboratory settings as a gelling reagent to provide a semi-solid surface for bacteria to grow on, often combined with liquid media that contain nutrients for encouraging bacterial growth, and is often made fresh when needed. However, typically, agar and agarose are only utilised in a hydrated state and is disposed of if it becomes dehydrated for any reason. By providing dehydrated agar or agarose as the dehydrated gel medium that forms part of the dehydrated bacterial growth platform of the present invention, the sample chamber may utilize a reliable bacterial growth platform in a manner that drastically extends the shelf life of the sample chamber.

In some examples, the dehydrated bacterial growth platform is provided as a film on an internal surface of the sample chamber.

The bacterial growth platform is where bacteria suspended in the fluid sample will begin to grow within the sample chamber. This is particularly true when the sample chamber includes an absorptive element adapted to expand and press a flexible filter, adapted to prevent bacteria from passing therethrough, against the bacterial growth platform.

As outlined above, imaging systems for capturing images of bacteria typically have very shallow depths of field, meaning that only shallow planes can be captured in focus by the system. By providing the bacterial growth platform as a film on a surface of the sample chamber, the bacterial growth is encouraged to occur on said film, meaning that the bacterial growth will occur on a thin, flat surface that can be readily imaged by a system having a shallow depth of field.

In some examples, the film has a thickness between 0.1 µm and 100 µm, for example a thickness between 0.5 µm and 75 µm, for example a thickness of 50 µm. As bacterial growth only occurs on the surface of a bacterial growth platform, a thin film of bacterial growth platform may be sufficient to encourage bacterial growth in the sample chamber whilst still maintaining a small form factor for the sample chamber itself.

In some examples, the film has a thickness of 100 µm on rehydration. For example, the dehydrated bacterial growth platform may have a thickness of 50 µm, and may then swell to a thickness of 100 µm on rehydration.

In some examples, the bacterial growth platform comprises a bacterial growth medium.

In this way, the bacterial growth platform may be provided with a bacterial growth medium including nutrients for encouraging bacterial growth on the bacterial growth platform.

In some examples, the sample chamber further comprises a partition dividing the sample chamber into an upstream sub-chamber and a downstream sub-chamber, wherein the downstream sub-chamber is fluidically connected to the upstream sub-chamber via the partition, and wherein the dehydrated bacterial growth platform is provided in the upstream sub-chamber.

For example, when the downstream sub-chamber contains an absorptive element as described above, the expansion of the absorptive element forces the partition into contact with the bacterial growth platform and clamps it flat in place in order to encourage bacterial growth in a given plane. Accordingly, the bacteria is located in the optimal place, for both encouraging bacterial growth and imaging the bacteria, in a manner that is automated entirely by the introduction of a fluid sample to the sample chamber and without requiring any active, or powered, components or any user input.

In some examples, the dehydrated bacterial growth platform is provided on an internal surface of the upstream sub-chamber opposite the partition. The bacterial growth platform may maintain a separation with the partition on rehydration in the absence of another force bringing them together, for example the expansion of the absorptive element. In some examples, the dehydrated bacterial growth platform is arranged parallel to the partition.

In this way, the contact between the partition and the bacterial growth platform may be controlled by the swelling of an absorptive element as described above. This may provide a means of achieving equal contact time between the bacteria captured by the partition and the bacterial growth platform across multiple sample chambers connected, for example, by a channel network as described above.

In some examples, the sample chamber further comprises a fluid inlet fluidically connected to the upstream sub-chamber, and wherein the fluid inlet is located adjacent the dehydrated bacterial growth platform. In this way, the fluid sample is introduced to the dehydrated bacterial growth platform immediately upon entry into the sample chamber. Accordingly, the process of rehydrating the bacterial growth platform may begin as the sample chamber starts filling and may be completed before bacterial growth may need to begin.

In some examples, the sample chamber further comprises an absorptive element adapted to absorb at least part of the fluid sample, wherein the absorptive element is provided in the downstream sub-chamber and adapted to reduce a humidity of the sample chamber to prevent rehydration of the dehydrated bacterial growth platform prior to contact with the fluid sample.

Put another way, in addition to the functions described above, the provision of an absorptive element within the sample chamber may prevent the premature rehydration of the dehydrated bacterial growth platform, i.e., prior to the introduction of the fluid sample into the sample chamber. Further, the absorptive element may maintain the dehydrated state of any other components, such as reagents, prior to the introduction of the fluid sample to the sample cartridge. In this way, the shelf life of the dehydrated bacterial growth platform, the sample chamber and the sample cartridge comprising said sample chamber may be extended.

According to a seventh aspect of the invention, there is provided a sample cartridge for processing a fluid sample, the sample cartridge comprising: a fluid pathway having an upstream end and a downstream end, wherein the fluid pathway is adapted to receive the fluid sample at the upstream end and route the fluid sample to the downstream end; and a sample chamber as discussed above, wherein the sample chamber is fluidically connected to the downstream end of the fluid pathway.

According to an eighth aspect of the invention, there is provided a method for encouraging bacterial growth from a fluid sample, the method comprising: providing the fluid sample to a dehydrated bacterial growth platform, thereby rehydrating the dehydrated bacterial growth platform to encourage bacterial growth.

According to a ninth aspect of the invention, there is provided a method for processing a fluid sample using a sample cartridge, the method comprising: providing the fluid sample to a fluid pathway of the sample cartridge at an upstream end of the fluid pathway; providing the fluid sample to a sample chamber fluidically connected to a downstream end of the fluid pathway by continuing to provide the fluid sample to the fluid pathway, wherein the sample chamber comprises a dehydrated bacterial growth platform and providing the fluid sample to the sample chamber comprises: providing the fluid sample to the dehydrated bacterial growth platform, thereby rehydrating the dehydrated bacterial growth platform to encourage bacterial growth.

Whilst the various aspects of the invention have been laid out separately above, it should be noted that any aspect of the invention outlined above may be combined with any other aspect of the invention outlined above. Put another way, there is provided a sample cartridge for processing a fluid sample according to any aspect, or combination of aspects, described above.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Figures 1a to 1c** show schematic representations of sample cartridges according to various aspects of the invention.
**Figures 2 to 7** illustrate the filling of the sample cartridge of Figure 1c with a fluid sample.
**Figure 8** shows a schematic representation of an analyte chamber of a sample cartridge according to an aspect of the invention.
**Figures 9 and 10** illustrate the passage of a fluid sample through the analyte chamber of Figure 8.
**Figure 11** shows a schematic representation of a sample chamber according to an aspect of the invention.
**Figures 12 to 17** illustrate the filling of the sample chamber of Figure 11 with a fluid sample.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

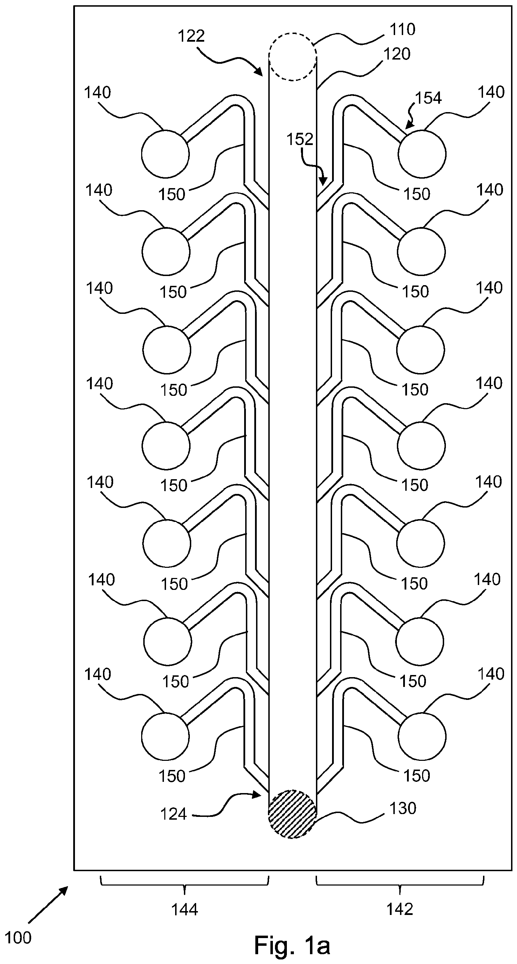

Figure 1a shows a schematic representation of a sample cartridge 100 for processing a fluid sample according to an aspect of the invention.

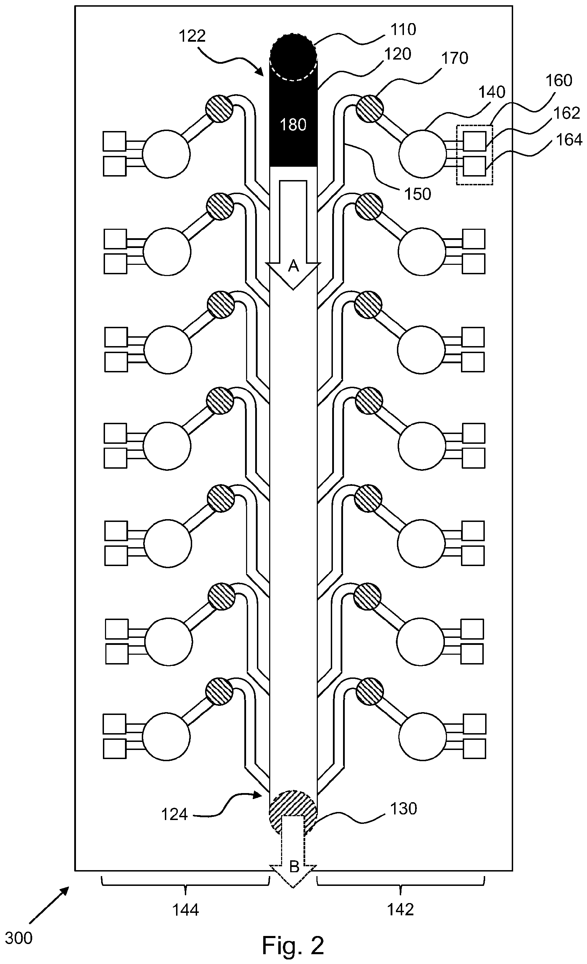

In the example shown in Figure 1a, the sample cartridge 100 includes an inlet 110 for receiving the fluid sample and a priming channel 120 having an upstream priming end 122 and a downstream priming end 124 opposite the upstream priming end 122. The priming channel includes a sealable vent in the form of a hydrophobic vent 130 provided at its downstream priming end 124 and the priming channel 120 is in fluid communication with the fluid inlet 110 at its upstream priming end 122. The hydrophobic vent 130 is gas permeable in a dry state. A fluid sample provided to the sample cartridge 100 at the fluid inlet 110 will flow along the priming channel 120 from the upstream priming end 122 towards the downstream priming end 124 and the hydrophobic vent 130 as described further below with reference to Figures 2 to 7.

The sample cartridge 100 includes a plurality of sample chambers 140, each of which is fluidically connected to the priming channel 120 by way of a branch channel 150. The branch channels 150 are each connected to the priming channel 120 at an upstream branch end 152 and are each connected to a respective one of the plurality of sample chambers 140 at a downstream branch end 154.

The upstream branch ends 152 of all of the branch channels 150 connecting the sample chambers 140 to the priming channel 120 are fluidically connected to the priming channel 120 between the upstream priming end 122 and the downstream priming end 124 of the priming channel 120.

In the example shown in Figure 1a, the plurality of sample chambers 140 are arranged into a first row 142 of sample chambers and a second row 144 of sample chambers. The first 142 and second 144 rows of sample chambers 140 are arranged on opposite sides of the priming channel 120.

Figure 1a shows the upstream branch ends 152 of the plurality of branch channels 150 fluidically connecting the first row 142 of sample chambers 140 to the priming channel 120 being aligned with the upstream branch ends 152 of the plurality of branch channels 150 fluidically connecting the second row 144 of sample chambers 140 to the priming channel 120. In particular, the upstream branch ends 152 are aligned in pairs along the length of the priming channel 120 of such that an upstream branch end 152 of a branch channel 150 connected to a sample chamber 140 in the first row of sample chambers 142 is arranged on the opposite side of the priming channel 120 to an upstream branch end 152 of a branch channel 150 connected to a sample chamber 140 in the second row of sample chambers 144.

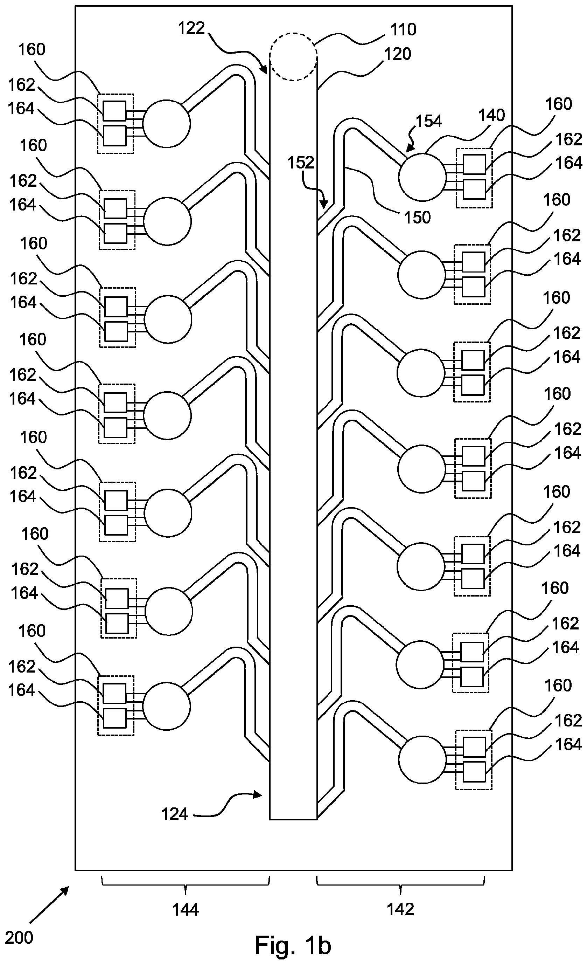

Figure 1b shows a schematic representation of a sample cartridge 200 for processing a fluid sample according to an aspect of the invention. Features in common with those present in Figure 1a share the same reference numerals.

In the example shown in Figure 1b, the sample cartridge 200 comprises a plurality of gas spring arrangements 160, each of which is fluidically connected to a respective one of the plurality of sample chambers 140. The gas spring arrangements 160 are connected downstream of the sample chambers 140 along the fluid pathway, the fluid pathway including the branch channel 150 connecting the sample chamber 140 to the priming channel 120 and the portion of the priming channel 120 connecting the branch channel 150 to the fluid inlet 110. The priming channel 120 may also be referred to as a main channel in the absence of the hydrophobic vent shown in Figure 1a.

Each gas spring arrangement 160 includes an upstream gas spring 162 fluidically connected to the sample chamber 140 at a first position and a downstream gas spring 164 fluidically connected to the sample chamber 140 at a second position different to the first position.

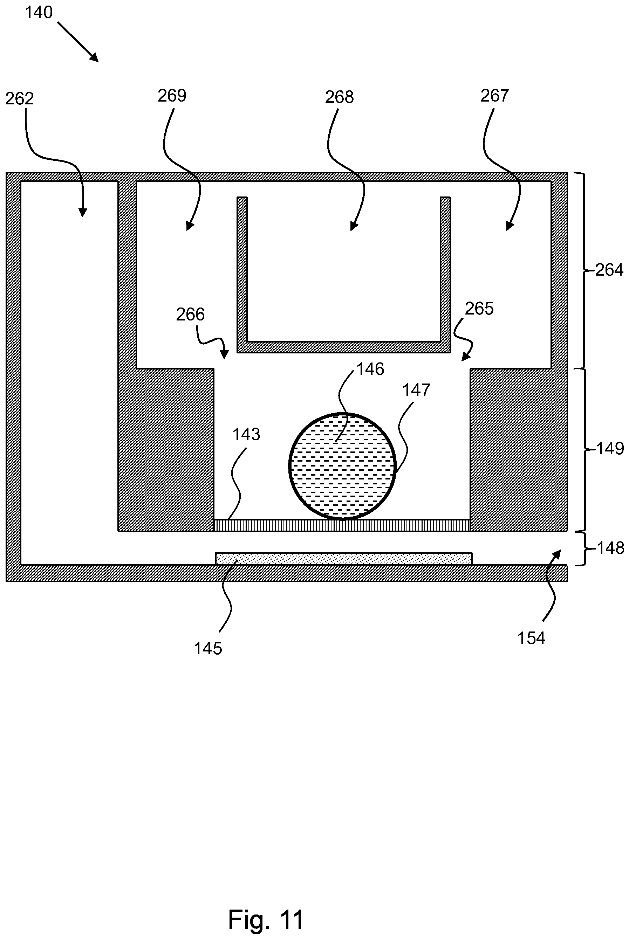

Exemplary gas spring arrangements 160, and in particular the two-stage gas spring arrangements 160 shown in Figure 1b, are described in further detail below with respect to Figures 11 to 17.

In the example shown in Figure 1b, the upstream branch ends 152 of the plurality of branch channels 150 fluidically connecting the first row 142 of sample chambers 140 to the priming channel 120 are offset, along the length of the priming channel 120, from the upstream branch ends 152 of the plurality of branch channels 150 fluidically connecting the second row 140 of sample chambers 140 to the priming channel 120. In the particular example shown in Figure 1b, the upstream branch ends 152 connecting the first row 142 of sample chambers 140 to the priming channel 120 are interleaved with the upstream branch ends 152 connecting the second row 144 of sample chambers 140 to the priming channel 120.

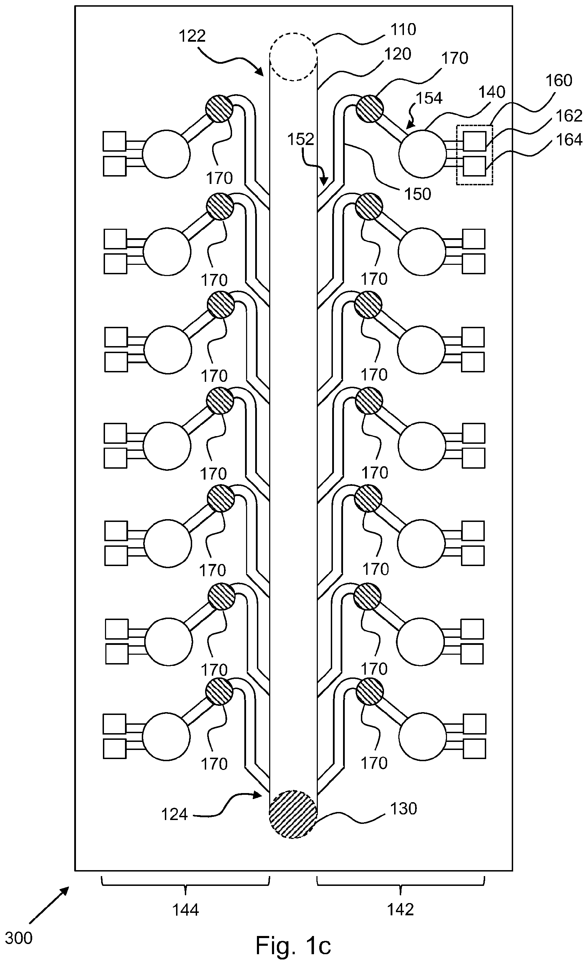

Figure 1c shows a schematic representation of a sample cartridge 300 for processing a fluid sample according to an aspect of the invention. Features in common with those present in Figures 1a and 1b share the same reference numerals.

The sample cartridge 300 shown in Figure 1c shares a combination of the features of the sample cartridges described above with reference to Figures 1a and 1b. In particular, the sample cartridge 300 of Figure 1c includes the hydrophobic vent 130 of Figure 1a and the gas spring arrangements of Figure 1b. Further, the downstream branch ends 152 of the branch channels 150 connecting the first 142 and second 144 rows of sample chambers 140 to the priming channel 120 of the sample cartridge 300 of Figure 1c are aligned as shown in Figure 1a.

In addition, each of the branch channels 150 of the sample cartridge 300 shown in Figure 1c comprises an analyte chamber 170 between the upstream branch end 152 and the downstream branch end 154. The analyte chambers 170 comprise a reagent to be mixed with the fluid sample as the fluid sample flows through the analyte chamber 170 from the priming channel 120 to the sample chamber 140.

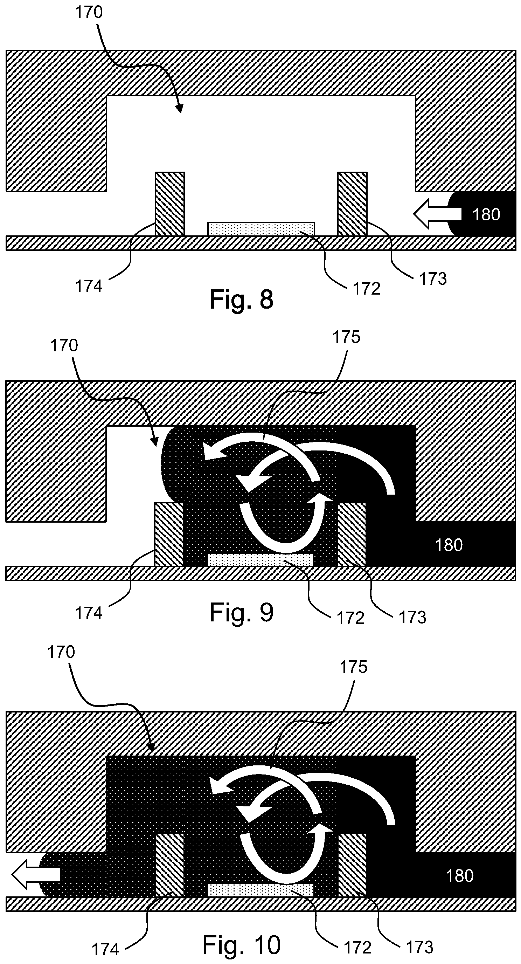

The contents of the analyte chamber 170 are discussed in further detail below with reference to Figures 8 to 10.

Figures 2 to 7 illustrate the filling of the sample cartridge 300 of Figure 1c with a fluid sample 180 intended for processing by the sample cartridge 300.

Figure 2 shows the sample cartridge 300 as the fluid sample 180 begins to enter the priming channel 120 from the fluid inlet 110. As the fluid sample 180 is introduced to the fluid inlet 110, the fluid sample 180 begins flowing from the upstream priming end 122 towards the downstream priming end 124 of the priming channel, as indicated by arrow A.

As the fluid sample 180 is introduced to the priming channel 120, via the fluid inlet 110, the air in the channel network is displaced and exits the sample cartridge 300 via the hydrophobic vent 130, as indicated by arrow B. The hydrophobic vent 130 will be dry at this stage and so will be gas permeable.

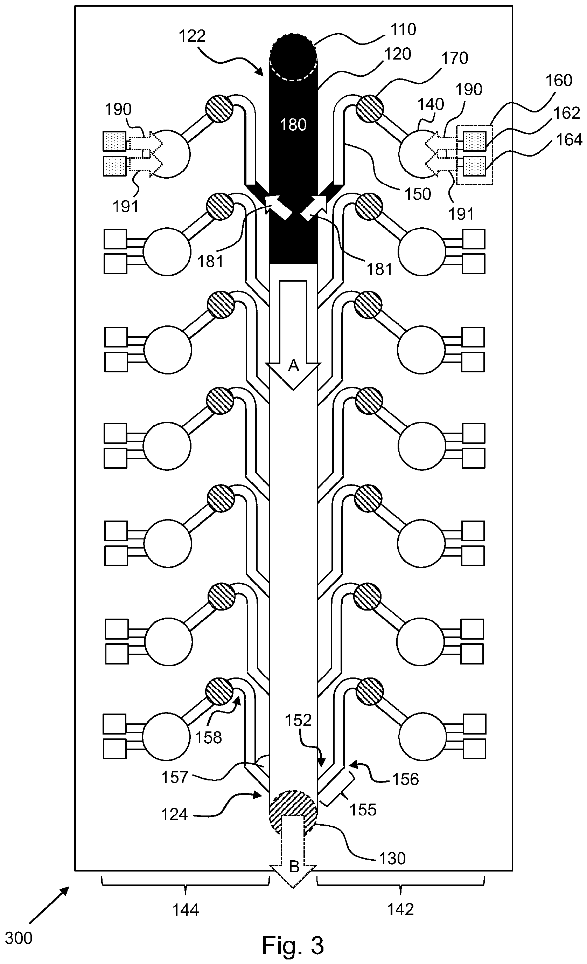

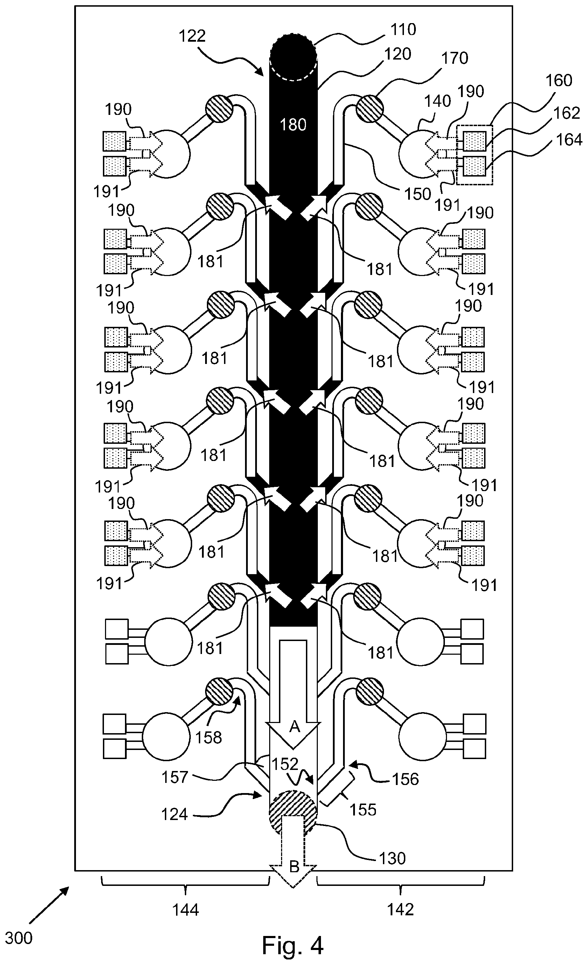

Figures 3 and 4 show the sample cartridge 300 as the fluid sample 180 continues along the priming channel 120 in direction A and past the downstream branch ends 152 of the branch channels 150 connecting the first 142 and second 144 rows of sample chambers 140 to the priming channel 120.

As the fluid sample 180 passes the downstream branch ends 152 of the branch channels 140, the branch channels 140, the sample chambers 150 and the gas spring arrangements 160 effectively become sealed as the only external air outlet in the sample cartridge 300 is the hydrophobic vent 130. Therefore, if a portion 181 of the fluid sample 180 flows along the branch channels 150, the air downstream of the portion 181 of the fluid sample 180 will be compressed and the pressure within the upstream gas spring 162 and the downstream gas spring 164 will increase. The increase in air pressure in the upstream gas spring 162 and the downstream gas spring 164 will exert a force 190, 191 against the portion 181 of the fluid sample 180 in the branch channel 150 and will prevent the fluid sample from continuing to flow along the branch channels 150 whilst the hydrophobic vent 130 remains dry and open to act as a pressure release.

In order to further guard against the fluid sample 180 progressing along the branch channels 150 too far, for example as far as the analyte chamber 170, each of the plurality of branch channels 150 comprises a branch inlet section 155 extending from the upstream branch end 152 of the branch channel to a downstream branch inlet end 156. The angle 157 between a priming flow vector, the priming flow vector being defined from the upstream priming end 122 to the downstream priming end 124 of the priming channel 120, and a branch flow vector, the branch flow vector being defined from the upstream branch end 152 to the downstream branch inlet end 156, is an acute angle.

Further, to prevent the analyte chamber 170 from being reached by the fluid sample 180 prematurely, the branch channels 150 may comprise at least one bend 158 between the upstream branch end 152 and the downstream branch end 154 of the branch channel 150. The branch channel 150 is routed to cause the fluid sample 180 in at least part of the branch channel 150 to flow in the opposite direction to the fluid sample 180 in the priming channel 120. As shown in Figures 1c to 7, the analyte chamber 170 is provided downstream of the bend 158.

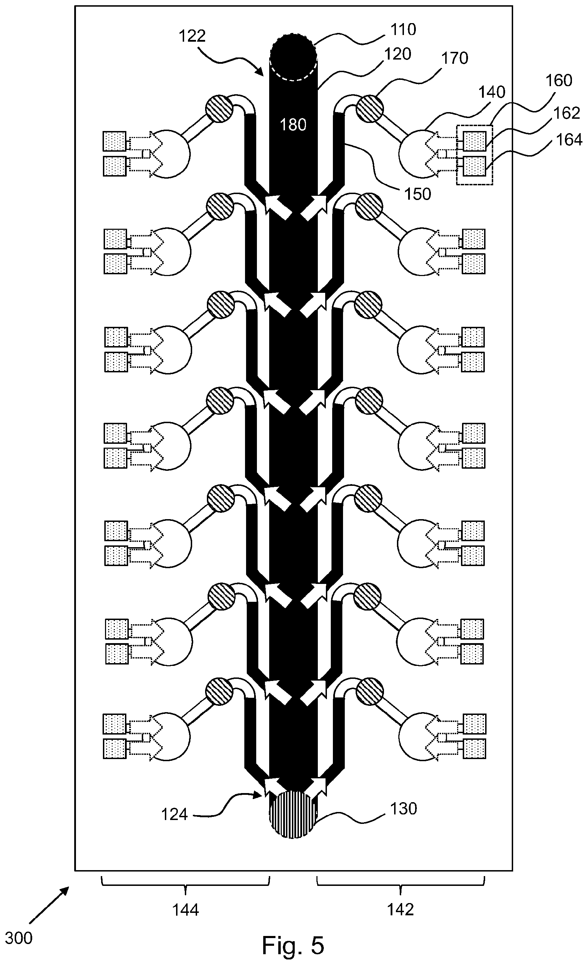

Figure 5 shows the sample cartridge 300 as the fluid sample 180 fills the priming channel 120 and wets the hydrophobic vent 130, thereby closing, or sealing, the hydrophobic vent 130.

Once the hydrophobic vent 130 has been wetted, and is no longer gas permeable, the sample cartridge 300 is essentially sealed. Therefore, any further fluid sample 180 introduced to the fluid inlet 110 will cause the fluid sample 180 to flow along the branch channels 150. As the filling of the branch channels has been arrested as the priming channel 120 fills by the pressure difference between the priming channel 120 and branch channels 150, due to the action of the gas spring arrangements 160 and the hydrophobic vent 130, all of the branch channels 150 will begin to fill simultaneously.

Further, as the gas spring arrangements, i.e., the upstream 162 and downstream 164 gas springs, connected to each sample chamber 140 are identical, the pressures in the gas spring arrangements 160 will tend towards equilibrium as the branch channels 150 continue to fill. This interaction between the gas spring arrangements 160 will force the branch channels 150 and the sample chambers 140 to fill with fluid sample 180 at the same rate.

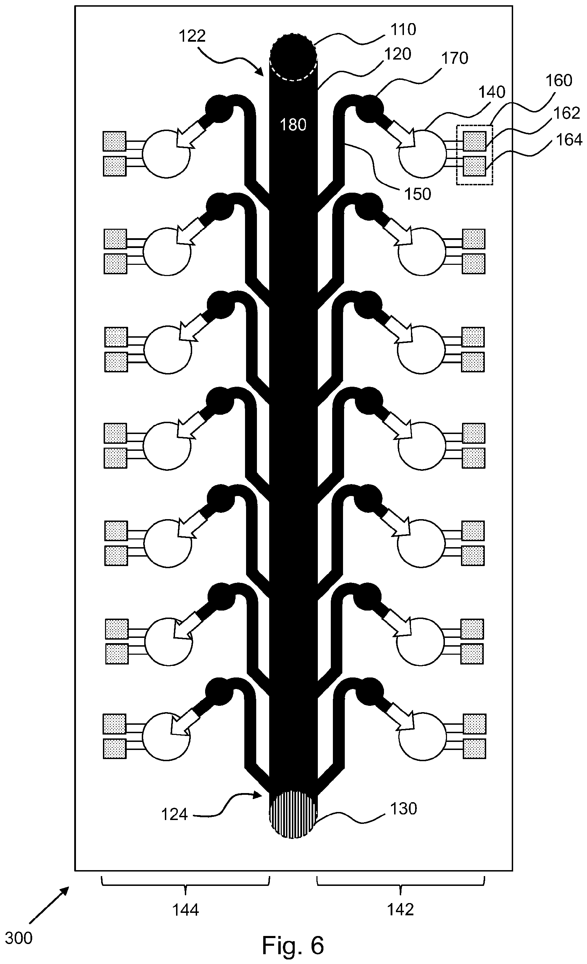

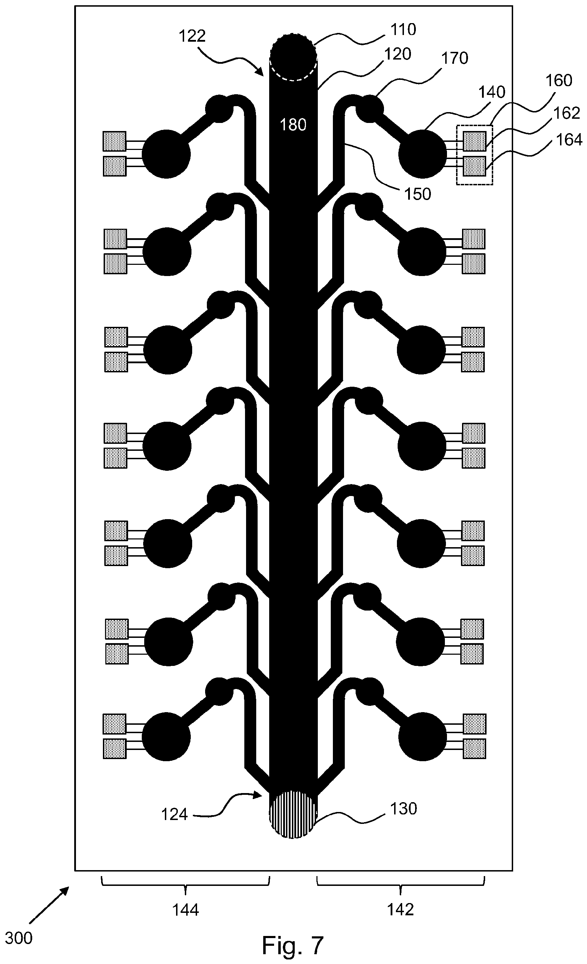

As shown in Figures 6 and 7, the features of the sample cartridge 300 described above, and in particular the interaction between the hydrophobic vent 130 and the gas spring arrangements 160, means that the fluid sample 180 in each branch channel 150 will reach each analyte chamber 170 and then each sample chamber 140 at substantially the same time. Thus, the sample cartridge 300 provides a means of achieving simultaneous filling of a plurality of sample chambers 140 controlled using only passive pressure controls throughout the sample cartridge 300 activated by the fluid sample itself.

Figures 8 to 10 illustrate the filling of the analyte chamber 170 of the sample cartridge 300 with the fluid sample 180. For example, Figures 8 to 10 illustrate the state of the analyte chamber 170 between the states of the sample cartridge 300 shown in Figures 5 to 7.

The analyte chamber 170 includes a reagent 172 to be mixed with the fluid sample 180 as the fluid sample flows through the analyte chamber. The analyte chamber 170 further comprises mixing structures 173, 174 adapted to generate turbulence 175 in a flow of fluid sample through the analyte chamber in order to improve the mixing of the reagent with the fluid sample 180.

As shown in Figures 8 to 10, as the fluid sample 180 flows into and through the analyte chamber 170, the mixing structures 173, 174 disrupt the flow of the fluid sample 180 in order to generate turbulence 175. The turbulent flow of the fluid sample 180 encourages the mixing of the reagent 172 with the fluid sample. The fluid sample 180 with the reagent 172 suspended therein continues to flow through the reagent chamber 170 and out towards the sample chamber 140.

Figure 11 shows a sample chamber 140 according to an aspect of the invention. The sample chamber 140 shown in Figure 11 may represent each of the plurality of sample chambers 140 shown in Figures 1 to 7.

The sample chamber 140 comprises an upstream sub-chamber 148 and a downstream sub-chamber 149 separated from each other, but fluidically connected via, a partition 143. The partition 143 is fluid permeable under a given pressure, meaning that below said given pressure the upstream 148 and downstream 149 sub-chambers are fluidically isolated from each other and above said given pressure upstream 148 and downstream 149 sub-chambers are fluidically connected to each other. The upstream sub-chamber 148 is fluidically connected to the downstream branch end 154 of the branch channel connecting the sample chamber 140 to the priming channel 120.

The upstream sub-chamber 148 is fluidically connected to a priming gas spring 262, which may be the upstream gas spring 162 described above with respect to Figures 1 to 7. The downstream sub-chamber 149 is fluidically connected to a balancing gas spring 264, which may be the downstream gas spring 164 described above with respect to Figures 1 to 7. The balancing gas spring 264 is fluidly connected to the downstream sub-chamber by a plurality of ducts 265, 266. The balancing gas spring comprises a plurality of gas spring chambers 267, 268, 269 fluidically connected to each other. Two of the gas spring chambers 267, 269 are directly fluidically connected to the downstream sub-chamber 144 via the ducts 265, 266 and one of the gas spring chambers 268 is only fluidically connected to the other gas spring chambers 267, 269.

In the example shown in Figure 11, the sample chamber includes a dehydrated bacterial growth platform 145, in the form of a combination of dehydrated agarose, adapted to rehydrate on contact with the fluid sample, and a dehydrated growth medium containing nutrients to encourage bacterial growth in the sample chamber.

Figure 11 shows the dehydrated bacterial growth platform 145 provided as a film on an internal surface of the sample chamber 140, and in particular an internal surface of the upstream sub-chamber 148 parallel to the partition 143. The dehydrated bacterial growth platform 154 has a thickness between 0.1 µm and 100 µm, for example a thickness between 0.5 µm and 75 µm, for example a thickness of 50 µm. The dehydrated bacterial growth platform 145 is arranged adjacent the fluid inlet of the upstream sub-chamber 148, i.e., the downstream branch end 154 of the branch channel.

In the example shown in Figure 11, the sample chamber 140 further comprises an absorptive element 146 adapted to absorb at least part of the fluid sample and adapted to reduce the humidity of the sample chamber 140 to prevent rehydration of the dehydrated bacterial growth platform 145 prior to contact with the fluid sample. In the particular example shown in Figure 11, the absorptive element 146 is a spherical ball of hydrogel movably received in the downstream sub-chamber 144 of the sample chamber 140. The absorptive element 146 comprises a dissolvable coating 147 to retard the absorption of the fluid sample by the absorptive element once it is introduced to the downstream sub-chamber 149.

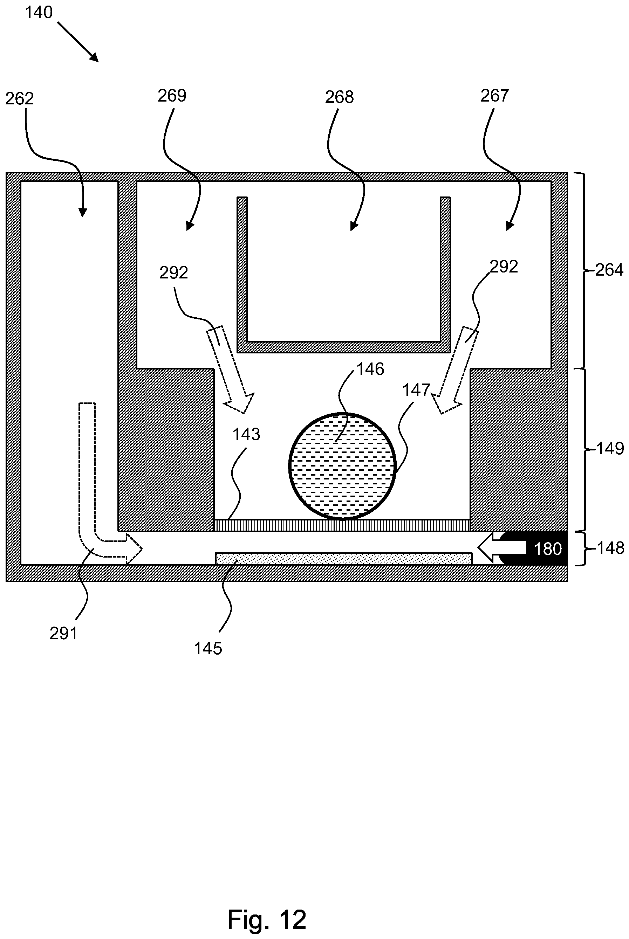

Figures 12 to 17 illustrate the filling of the sample chamber 140 shown in Figure 11. As discussed above, the filling procedure illustrated in Figures 12 to 17 may occur in all of the sample chambers 140 of the sample cartridges shown in Figures 1a to 7 simultaneously.

The fluid sample 180 enters the upstream sub-chamber 148, at which point the entire branch channel 150 leading to the sample chamber will have been filled, for example as shown in Figure 6. The air pressure in the priming gas spring 262 and the balancing gas spring 264 will have been building as the branch channel 150 was filling with the fluid sample and forces 291 and 292 will be acting against the movement of the fluid sample into the sample chamber.

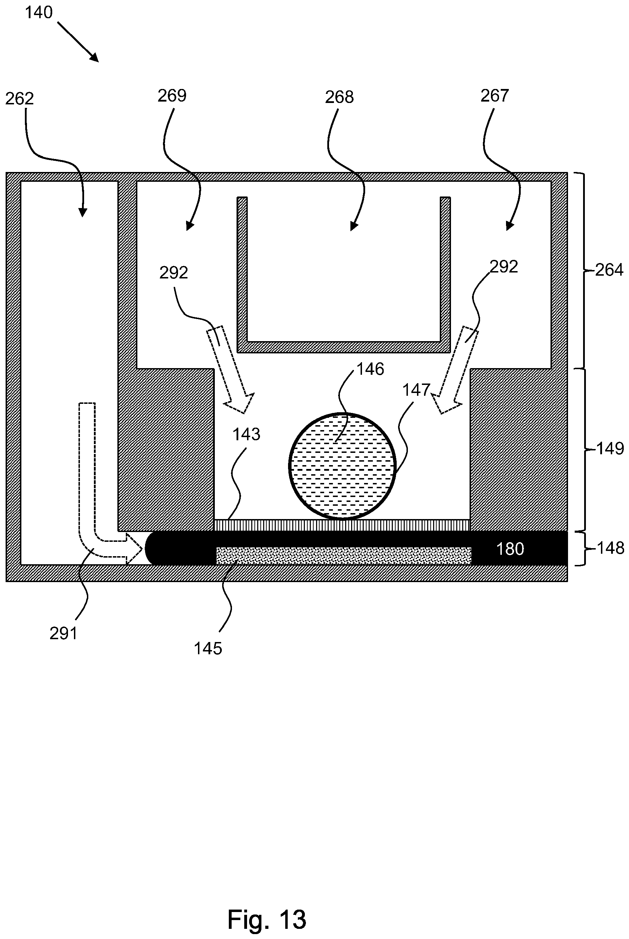

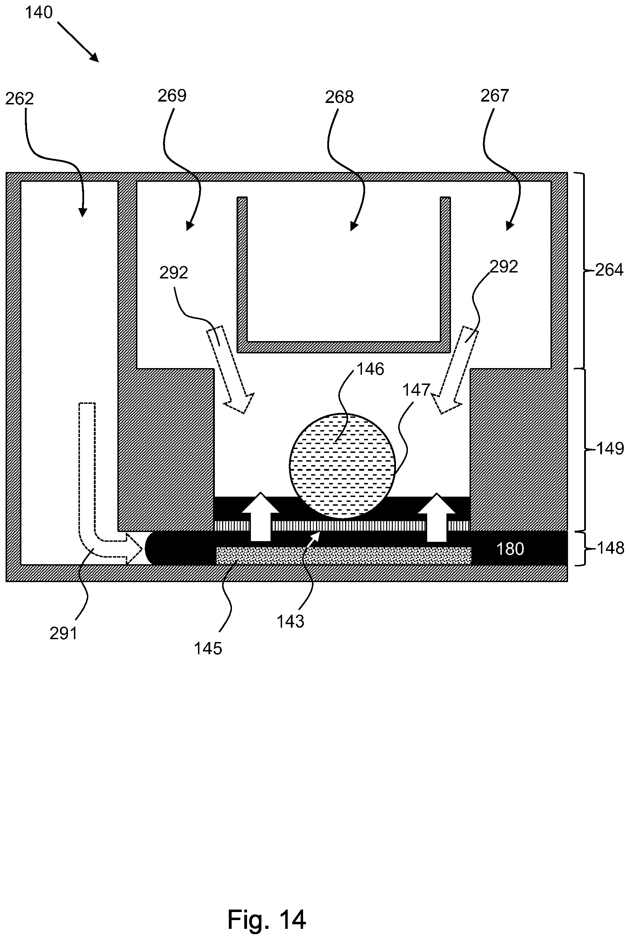

The fluid sample 180 fills the upstream sub-chamber 148 as shown in Figure 13 until the pressure in the priming gas spring 262 reaches the threshold pressure to cause the fluid sample to pass through the partition and into the downstream sub-chamber as shown in Figure 14.

As the fluid sample 180 comes into contact with the dehydrated bacterial growth platform 145, it rehydrates to form a bacterial growth platform suitable for encouraging the growth of bacteria thereon. The bacterial growth platform may swell on rehydration, for example to a thickness of 100 µm from a dehydrated thickness of 50 µm. Despite the swelling, the bacterial growth platform maintains a separation from the partition 143 in order to permit the free flow of fluid sample through the partition 143 without blocking the fluid pathway.

The partition 143 shown in Figures 11 to 17, comprises a fluid permeable filter adapted to prevent the passage of bacteria through the filter. Therefore, as the fluid sample 180 passed through the partition from the upstream sub-chamber 148 to the downstream sub-chamber 149, bacteria contained within the fluid sample will be left on the partition.

Further, as the fluid sample 180 enters the downstream sub-chamber 149, the fluid sample 180 contacts the absorptive element 146 and begins to dissolve the dissolvable coating 147 as the sample chamber 140 continues to fill with the fluid sample.

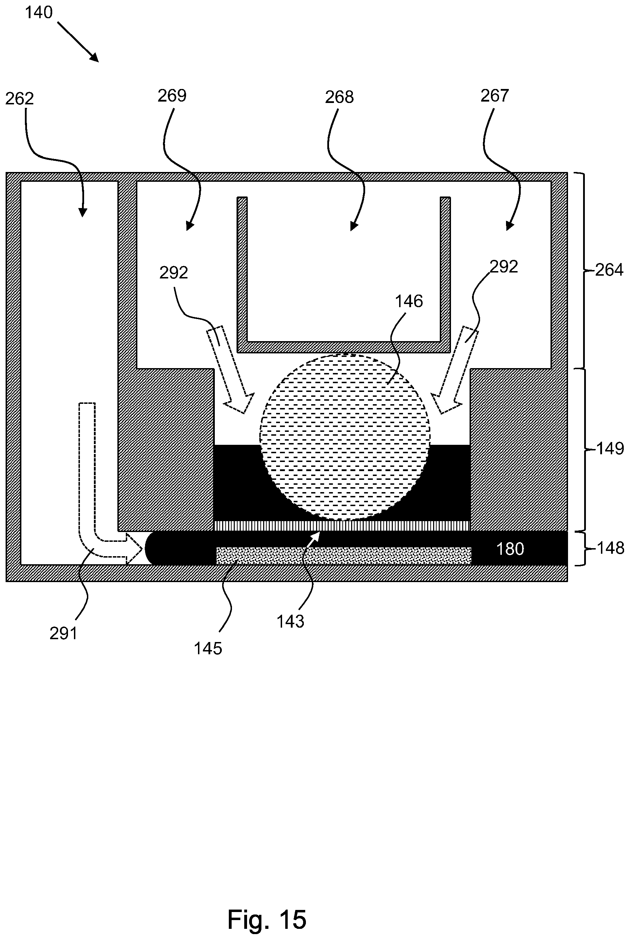

As shown in Figure 15, once the dissolvable coating 147 of the absorptive element 146 has been dissolved, the absorptive element 146 begins to absorb the fluid sample 180. In the absence of the fluid sample 180 in the downstream sub-chamber 144, the absorptive element 146 is in a dehydrated state (as shown in Figure 12), and when the absorptive element 146 absorbs at least part of the fluid sample 180 it changes from the dehydrated state to a hydrated state. The volume of the absorptive element 146 in the hydrated state (as shown in Figures 15 to 17) is larger than a volume of the absorptive element 146 in the dehydrated state (as shown in Figures 12 to 14).

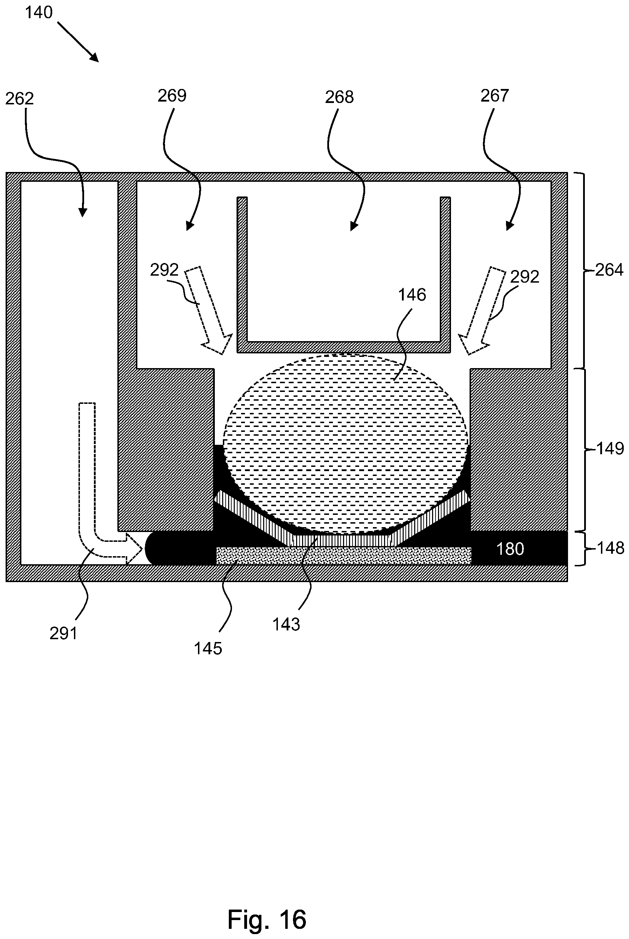

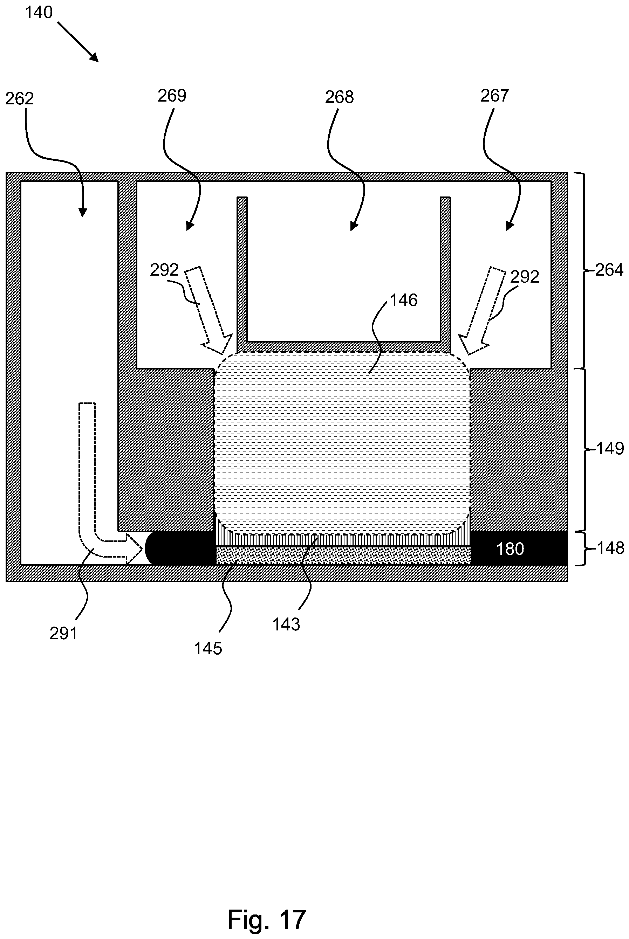

As shown in Figures 16 and 17, the volume of the downstream sub-chamber 149 is lower than the volume of the absorptive element 146 in the hydrated state and as the absorptive element 146 continues to swell, the absorptive element 146 exerts a force on the partition 143. The partition 143 being a flexible bacterial filter, the partition deforms under the force exerted by the expanding absorptive element 146 until the partition is flattened against the bacterial growth platform 145.

As the lower surface of the partition 143 is covered in bacteria captured from the fluid sample flowing through the partition 143 from the upstream sub-chamber 148 to the downstream sub-chamber 149, the bacteria will be brought into contact with the bacterial growth platform 145. Further, as the partition 143 is flattened against the bacterial growth platform 145, the bacteria growing on the bacterial growth platform will all be constrained to the same plane, which enables accurate imaging and analysis of the bacteria within the sample chamber.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A sample cartridge for processing a fluid sample, the sample cartridge comprising:
a fluid pathway having an upstream end and a downstream end, wherein the fluid pathway is adapted to receive the fluid sample at the upstream end and route the fluid sample to the downstream end;
a sample chamber fluidically connected to the downstream end of the fluid pathway, the sample chamber comprising:
a partition dividing the sample chamber into an upstream sub-chamber and a downstream sub-chamber, wherein the upstream sub-chamber is fluidically connected to the downstream end of the fluid pathway, and wherein the downstream sub-chamber is fluidically connected to the upstream sub-chamber via the partition; and
an absorptive element adapted to absorb at least part of the fluid sample, wherein the absorptive element is provided in the downstream sub-chamber.

2. The sample cartridge claimed in claim 1, wherein:
in the absence of the fluid sample in the downstream sub-chamber, the absorptive element is in a dehydrated state; and
in the presence of the fluid sample in the downstream sub-chamber, the absorptive element is adapted to absorb at least part of the fluid sample to change from the dehydrated state to a hydrated state, and
wherein, a volume of the absorptive element in the hydrated state is larger than a volume of the absorptive element in the dehydrated state.

3. The sample cartridge claimed in claim 2, wherein the absorptive element is movably received in the downstream sub-chamber when the absorptive element is in the dehydrated state.

4. The sample cartridge claimed in any of claims 2 to 3, wherein a volume of the downstream sub-chamber is lower than the volume of the absorptive element in the hydrated state, such that the absorptive element in the hydrated state exerts a force on the partition, and optionally wherein the partition is flexible such that the partition deforms under the force exerted by the absorptive element in the hydrated state.

5. The sample cartridge as claimed in any of claims 1 to 4, wherein the partition is adapted to become transparent on contact with the fluid sample.

6. The sample cartridge claimed in any of claims 1 to 5, wherein the upstream sub-chamber comprises a bacterial growth platform.

7. The sample cartridge claimed in claim 6, when dependent on claim 5, wherein a volume of the sample chamber is lower than the volume of the absorptive element in the hydrated state, such that the partition is deformed to contact the bacterial growth platform.

8. The sample cartridge claimed in claim 7, wherein the partition is deformed to be substantially flattened against the growth medium.

9. The sample cartridge claimed in any preceding claim, wherein the absorptive element is three-dimensional, and optionally wherein the absorptive element is spherical.

10. The sample cartridge claimed in any preceding claim, wherein the absorptive element comprises a hydrogel.

11. The sample cartridge claimed in any preceding claim, wherein the absorptive element comprises a dissolvable coating to adjust an absorption rate of the fluid sample by the absorptive element.

12. The sample cartridge claimed in any preceding claim, wherein the partition comprises a fluid permeable filter adapted to prevent the passage of bacteria through the filter, the bacteria being present in the fluid sample.

13. The sample cartridge claimed in any preceding claim, wherein the fluid pathway widens along a downstream direction as it approaches the sample cartridge from an upstream direction.

14. A method for filling a sample chamber of a sample cartridge with a fluid sample, the sample chamber comprising a partition dividing the sample chamber into an upstream sub-chamber and a downstream sub-chamber, and wherein the sample chamber comprises an absorptive element provided in the downstream sub-chamber adapted to absorb at least part of the fluid sample, the method comprising:
providing a fluid sample to a fluid pathway of the sample cartridge, thereby filling an upstream sub-chamber of the sample chamber that is fluidically connected to a downstream end of the fluid pathway; and
continuing to provide the fluid sample to the fluid pathway of the sample cartridge, thereby causing the fluid sample to flow through the partition from the upstream sub-chamber to the downstream sub-chamber.

15. The method of claim 14, wherein the partition is flexible and the upstream sub-chamber comprises a bacterial growth platform, and wherein the method further comprises:
deforming the partition to contact the bacterial growth platform, wherein deforming the partition comprises:
continuing to provide the fluid sample to the fluid pathway of the sample cartridge, thereby causing the fluid sample to be absorbed by the absorptive element, such that the absorptive element changes from a dehydrated state to a hydrated state, wherein a volume of the absorptive element in the hydrated state is larger than a volume of the sample chamber such that the absorptive element in the hydrated state exerts a force on the partition, thereby deforming the partition to contact the bacterial growth platform.
